(19) Europäisches Patentamt
European Patent Office
Office européen des brevets

(11) EP 0 839 520 B1

(12) **FASCICULE DE BREVET EUROPEEN**

(45) Date de publication et mention de la délivrance du brevet:
**07.01.1999 Bulletin 1999/01**

(51) Int. Cl.$^6$: **A61K 7/48**, A61K 7/50

(21) Numéro de dépôt: **97402297.2**

(22) Date de dépôt: **01.10.1997**

(54) **Composition rinçable pour le soin de la peau**

Zusammensetzung zur Pflege der Haut, die abgespuelt werden kann

Rinsing composition for the care of the skin

(84) Etats contractants désignés:
**DE ES FR GB IT**

(30) Priorité: **04.11.1996 FR 9613404**
**16.04.1997 FR 9704692**

(43) Date de publication de la demande:
**06.05.1998 Bulletin 1998/19**

(73) Titulaire: **L'OREAL**
**75008 Paris (FR)**

(72) Inventeurs:
- **Simon, Pascal**
  **94400 Vitry/S/Seine (FR)**
- **Bordeaux, Dominique**
  **91240 Saint Michel Sur Orge (FR)**
- **Afriat, Isabelle**
  **75014 Paris (FR)**

(74) Mandataire:
**Tezier Herman, Béatrice**
**L'OREAL,**
**Département Propriété Industrielle,**
**90, rue du Gal Roguet**
**92583 Clichy Cédex (FR)**

(56) Documents cités:

**EP-A- 0 274 812**
**EP-A- 0 588 379**
**WO-A-92/07543**
**WO-A-93/08840**
**WO-A-94/17830**
**DE-A- 4 139 935**
**FR-A- 2 237 615**
**US-A- 4 268 498**
**US-A- 4 482 537**
**US-A- 4 687 843**

- **CHEMICAL ABSTRACTS, vol. 93, no. 18, 3 novembre 1980 Columbus, Ohio, US; abstract no. 173598z, page 345; colonne I; XP002029045 & COSMET.TOILETRIES, vol. 95, no. 3, 1980, pages 85-86,**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 839 520 B1

## Description

L'invention se rapporte à une composition ayant l'aspect d'un gel transparent rinçable pour son utilisation comme vecteur d'actifs cosmétiques et/ou dermatologiques.

L'invention se rapporte également aux compositions cosmétiques et/ou dermatologiques comprenant un tel vecteur et au moins un actif cosmétique et/ou dermatologique, choisi par exemple parmi les actifs dépigmentants, kératolytiques, amincissants, tenseurs, de traitement de l'acné, anti-inflammatoires, auto-bronzants.

Les actifs cosmétiques et/ou dermatologiques sont habituellement appliqués sur la peau dans des compositions dans lesquelles ils ont été incorporés, ces compositions se trouvant sous la forme de crèmes, de laits, de gels, que l'on étale sur la peau en massant du bout des doigts pour favoriser la pénétration de la composition, et en particulier des actifs, dans la peau. Ces compositions sont habituellement appliquées après un nettoyage de la peau, et on évite de rincer la peau après leur application pour obtenir une bonne efficacité des actifs cosmétiques et/ou dermatologiques.

Ce mode d'application d'actifs cosmétiques et/ou dermatologiques n'est pas dépourvu d'inconvénients : les véhicules cosmétiques et/ou dermatologiques habituels comprennent différents ingrédients cosmétiques, dont notamment des tensioactifs et des conservateurs, en quantités non négligeables, à la fois pour stabiliser le véhicule cosmétique, pour y dissoudre l'actif et/ou pour protéger la composition d'éventuelles contaminations microbiennes. L'application répétée et prolongée de tels milieux peut induire une sensibilisation de la peau et provoquer des réactions d'intolérance.

Pour solubiliser les actifs, on utilise habituellement dans les produits de soin des milieux comprenant des corps gras et/ou des glycols qui confèrent à ces compositions une certaine lourdeur. Or, certains utilisateurs trouvent désagréable de conserver sur le visage pendant plusieurs heures, en règle générale la journée, un produit de soin, ces produits étant parfois jugés gras ou collants.

Il subsiste donc le besoin d'une composition pouvant être utilisée comme véhicule pour des actifs cosmétiques et/ou dermatologiques, cette composition étant rinçable, stable, douce pour la peau et ayant la propriété de potentialiser les actifs de telle sorte qu'après un court temps d'application suivi d'un rinçage. on obtienne la même efficacité de traitement non thérapeutique de la peau qu'avec un produit non rincé, et une efficacité supérieure aux compositions rincées de l'art antérieur.

De façon étonnante, la demanderesse a découvert des compositions pouvant être utilisées comme vecteur d'actifs cosmétiques et/ou dermatologiques, ces compositions étant rinçables et présentant une efficacité comparable après un court temps d'application, suivi d'un rinçage, à celle d'un vecteur cosmétique et/ou dermatologique non rincé habituel et supérieure à celle des produits rincés de l'art antérieur.

Aussi, l'invention a pour objet une composition cosmétique et/ou dermatologique comprenant au moins un actif cosmétique et/ou dermatologique caractérisée en ce que cet actif est dissous ou dispersé dans un vecteur ayant l'aspect d'un gel transparent stable, comprenant :

(i) au moins 20 % d'une phase grasse,
(ii) au moins un ester gras d'hydrate de carbone en $C_5$-$C_7$,
(iii) au moins un polyol.

Le mot « transparent » signifie qu'au travers d'une bouteille transparente contenant la composition on peut distinguer les caractères imprimés sur une page de journal placée derrière cette bouteille.

La viscosité des compositions selon l'invention est, à température ambiante (25°C) et à pression normale, préférentiellement supérieure à 2 Pa.s, plus préférentiellement à 4 Pa.s et encore plus préférentiellement à 5 Pa.s. De préférence elle est inférieure à 25 Pa.s

De préférence, la composition selon l'invention comprend en outre de l'eau. Dans ce cas elle est avantageusement sous la forme d'une émulsion huile-dans eau ayant l'aspect d'un gel.

Par hydrates de carbone en $C_5$-$C_7$, on entend des pentoses, hexoses et heptoses (non réduits) et leurs dérivés alkyl-olosides dont le groupement alkyle contient de 1 à 6 atomes de carbone. Avantageusement, on choisit les hydrates de carbone parmi ceux ayant une chaîne à 6 atomes de carbone, et préférentiellement ils sont choisis parmi le glucose, le fructose et leurs dérivés alkyl-glucoside et alkyl-fructoside en $C_1$-$C_6$. Encore plus préférentiellement, l'hydrate de carbone est le glucose ou un dérivé alkyl-glucoside, comme par exemple le méthyl-1-glucoside.

Par esters gras d'hydrates de carbone en $C_5$-$C_7$, on entend de préférence les composés obtenus par réaction d'un acide gras à chaîne saturée ou insaturée ayant de 8 à 30 atomes de carbone, préférentiellement de 12 à 22 atomes de carbone et encore plus préférentiellement de 16 à 20 atomes de carbone avec un hydrate de carbone choisi parmi les pentoses, les hexoses, les heptoses et leurs dérivés alkyl oloside dont le groupement alkyle contient de 1 à 6 atomes de carbone.

L'ester d'acide gras et d'hydrate de carbone peut contenir un mélange de dérivés mono-, di-, tri-, tétra-ester.

Avantageusement, les esters d'hydrates de carbone en $C_5$-$C_7$ sont oxyalkylénés. Préférentiellement les esters

d'hydrates de carbone en $C_5$-$C_7$ sont éthérifiés par un ou plusieurs fragments oxyéthylène ou oxypropylène, l'ensemble des substituants oxyéthylène ou oxypropylène représentant au total de 5 à 200, et préférentiellement 15 à 150, unités oxyde d'alkylène.

Avantageusement toutes les fonctions hydroxyle des dérivés d'hydrate de carbone utilisés dans les compositions selon l'invention sont substituées par un groupement ester ou par un groupement oxyde d'alkylène.

De tels composés sont bien connus de l'homme du métier. Plusieurs de ces composés sont disponibles commercialement. Par exemple on peut utiliser les dérivés vendus sous la marque GLUCAMATE par la société AMERCHOL.

Avantageusement les esters d'hydrate de carbone en $C_6$ sont choisis parmi les composés répondant à la formule (I) :

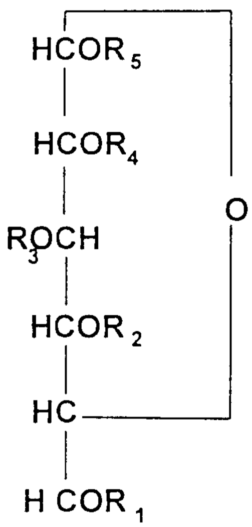

(I)

dans laquelle :

$R_1$ est un groupement de formule (III) :

$$-OC-R' \qquad (III)$$

R' étant choisi parmi les groupements alkyle linéaires ou ramifiés, saturés ou insaturés en $C_8$-$C_{30}$ et préférentiellement en $C_{12}$-$C_{22}$,

$R_2$, $R_3$, $R_4$, identiques ou différents, représentent un groupement choisi parmi :

- un atome d'hydrogène,
- un groupement de formule (II) :

$$-(C_nH_{2n}O)_p-R \qquad (II)$$

avec n=2 ou 3 ; p est un entier allant de 2 à 50; R représente un atome d'hydrogène ou un groupement alkyle en $C_1$-$C_6$,
- un groupement de formule (III) :

$$-OC-R' \qquad (III)$$

R' étant choisi parmi les groupements alkyle linéaires ou ramifiés, saturés ou insaturés en $C_8$-$C_{30}$ et préférentiellement en $C_{12}$-$C_{22}$,
l'un au moins de $R_2$, $R_3$, $R_4$ étant un groupement de formule (II), le total des résidus oxyalkylénés, $\Sigma p$, étant compris entre 5 et 200,

$R_5$ étant un groupement alkyle en $C_1$-$C_6$, linéaire ou ramifié, saturé ou insaturé.

De préférence, on choisit :

- $R_5 = CH_3$,
- au moins deux groupements parmi $R_2$, $R_3$, $R_4$, répondent à la formule (II),
- $\Sigma p$ est compris entre 15 et 150,
- R=H,
- au moins un groupement parmi $R_1$, $R_2$, $R_3$, $R_4$, répond à la formule (III),
- R' est choisi parmi les groupements alkyle en $C_{16}$-$C_{20}$,
- aucun des groupements $R_2$, $R_3$, $R_4$, n'est l'atome d'hydrogène.

Avantageusement, les compositions selon l'invention comprennent, en poids par rapport au poids total de la composition, de 0,5 à 50% d'ester d'hydrate de carbone en $C_5$-$C_7$ et préférentiellement de 2 à 20%.

Les compositions selon l'invention comprennent au moins un polyol. Ce polyol peut éventuellement être oxyalkylé. Avantageusement, le polyol comprend au moins deux fonctions hydroxyle libres. Ce polyol peut être choisi parmi l'éthylène glycol, le propylène glycol, le 1,3-butylène glycol, le dipropylène glycol, la glycérine, les polyglycérines, comme la diglycérine, la triglycérine et la tétraglycérine, le glucose, le maltose, le maltitol, le saccharose, le fructose, le sorbitol, les sucres issus de la décomposition de l'amidon et leurs mélanges.

Le polyol représente avantageusement de 0,5 à 60% en poids par rapport au poids total de la composition, de préférence de 2 à 40% et encore plus préférentiellement de 5 à 30%.

La nature de la phase grasse rentrant dans la composition des émulsions selon l'invention n'est pas critique; elle peut ainsi être constituée par tous les composés qui sont déjà connus de façon générale comme convenant pour la fabrication d'émulsions de type huile-dans-eau. En particulier, ces composés peuvent être choisis, seuls ou en mélanges, parmi les différents corps gras, les huiles d'origine végétale, animale ou minérale, les cires naturelles ou synthétiques, et analogues.

On peut citer parmi les huiles utilisables dans la présente invention les huiles d'origine végétale ou animale, comme par exemple le squalane, l'huile de coprah, l'huile de macadamia, l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide ; des huiles d'hydrocarbures telles que des huiles de paraffine, les isoparaffines, la vaseline ; des huiles de silicone comme les polydiméthylsiloxanes, les cyclopolydiméthylsiloxanes, les polyméthylphénylsiloxanes, des polysiloxanes modifiés par des acides gras, des polysiloxanes modifiés par des alcools gras, des polysiloxanes modifiés par des polyoxyalkylènes, des silicones fluorées ; des huiles perfluorées et/ou organofluorées ; des acides gras supérieurs tels que l'acide myristique, l'acide palmitique, l'acide stéarique, l'acide béhénique, l'acide oléique, l'acide linoléique, l'acide linolénique, l'acide isostéarique, des alcools gras supérieurs tels que le cétanol, l'alcool stéarylique, l'alcool oléique ; des mono et des di-esters parmi lesquels on peut citer en particulier le myristate d'isopropyle, le palmitate d'isopropyle, le stéarate de butyle, le laurate d'hexyle, l'isononanoate d'isononyle, le palmitate de 2-éthyl hexyle, le laurate de 2-hexyl décyle, le palmitate de 2-octyl décyle, le myristate de 2-octyl dodécyle, le succinate de 2-diéthylhexyle, le malate de diisostéaryle, le lactate de 2-octyl dodécyle, le triisostéarate de glycérine, l'adipate de di-n-butyle, l'adipate de di-(2-éthyl hexyle), le dioléate d'éthylène glycol, le di-isotridécanoate d'éthylène glycol, le di-isostéarate d'éthylène glycol, le di-caprylate de néopentylglycol.

De préférence on utilise une huile ou un mélange d'huiles ayant un indice de réfraction $^{20}n_D \leq 1{,}45$.

La phase grasse peut représenter de 20 à 95% en poids du poids total de la composition, de préférence de 20 à 85% et encore plus préférentiellement de 40 à 80%.

L'eau représente de préférence de 0,01 à 30% en poids par rapport au poids total de la composition. Avantageusement, l'eau représente de 2 à 20% en poids par rapport au poids total de la composition.

Habituellement on entend par eau de l'eau pure. Toutefois, une partie de l'eau utilisée dans les compositions selon l'invention peut éventuellement être choisie parmi les eaux minérales ou thermales. En général, une eau minérale est propre à la consommation, ce qui n'est pas toujours le cas d'une eau thermale. Chacune de ces eaux contient, entre autre, des minéraux solubilisés et des oligo-éléments. Ces eaux sont connues pour être employées à des fins de traitement spécifique selon les oligo-éléments et les minéraux particuliers qu'elles contiennent, tels que l'hydratation et la désensibilisation de la peau ou le traitement de certaines dermatoses. Par eaux minérales ou thermales, on désignera non seulement les eaux minérales ou thermales naturelles, mais également des eaux minérales ou thermales naturelles enrichies en constituants minéraux et/ou en oligo-éléments supplémentaires, ainsi que des solutions aqueuses minérales et/ou oligo-élémentaires préparées à partir d'eau purifiée (déminéralisée ou distillée). Une eau thermale ou minérale naturelle utilisée selon l'invention peut, par exemple être choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevard-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Uriage-les-bains, l'eau d'Avenne.

Les compositions cosmétiques de l'invention peuvent, en outre, contenir des adjuvants hydrosolubles ou liposolubles habituels dans le domaine cosmétique tels que les conservateurs, les antioxydants, les parfums, les filtres, les matières colorantes, les nacres.

Les compositions selon l'invention comprennent en outre au moins un actif cosmétique et/ou dermatologique, choisi par exemple parmi les actifs anti-rides, anti-vieillissement, hydratant, dépigmentants, kératolytiques, amincissants, tenseurs, de traitement de l'acné, antiséborrhéiques, anti-inflammatoires, autobronzants, antiradicaux libres, raffermissants, les actifs traitant des maladies de peau comme les mycoses, les dermites, le psoriasis. Suivant sa nature, hydrophile ou lipophile, cet actif est présent dans la phase aqueuse ou la phase grasse. De préférence ces actifs sont introduits dans le vecteur à raison de 0,1 à 10% en poids par rapport au poids total de la composition.

Parmi les actifs utilisables dans la présente invention, on peut citer par exemple : l'acide ascorbique et ses esters, l'allantoïne, l'acide citrique, l'acide caféique, l'acide salicylique et ses dérivés (par exemple acide n-octanoyl-5 ou décanoyl-5-salicylique), les $\alpha$-hydroxyacides tels que l'acide lactique, l'acide méthyllactique, l'acide glucuronique, l'acide glycolique, l'acide pyruvique, l'acide 2-hydroxy-butanoïque, l'acide 2-hydroxypentanoïque, l'acide 2-hydroxyhexanoïque, l'acide 2-hydroxyheptanoïque, l'acide 2-hydroxyoctanoïque, l'acide 2-hydroxy-nonanoïque, l'acide 2-hydroxydécanoïque, l'acide 2-hydroxyundéca noïque, l'acide 2-hydroxydodécanoïque, l'acide 2-hydroxytétradécanoïque, l'acide 2-hydroxyhexadécanoïque, l'acide 2-hydroxyoctadécanoïque, l'acide 2-hydroxy-tétraéco sanoïque, l'acide 2-hydroxyeïcosanoïque, l'acide mandélique, l'acide benzoïque, l'acide phényllactique, l'acide gluconique, l'acide galacturonique, l'acide aleuritique, l'acide ribonique, l'acide tartronique, l'acide tartrique, l'acide malique, l'acide fumarique, l'acide rétinoïque et ses dérivés, l'acide benzène 1,4-di(3-méthylidène 10-camphosulfonique), la dihydroxyacétone (DHA), les vitamines hydrosolubles, l'amidon, les extraits bactériens ou végétaux, notamment d'Aloe Vera, le tocophérol (vitamine E) et ses dérivés, les acides gras essentiels, les céramides, les huiles essentielles. On peut aussi, utiliser tous les composés naturels ou synthétiques contenant de tels acides, comme les extraits végétaux et plus spécialement les extraits de fruits. On peut utiliser les protéines végétales et leurs hydrolysats. On peut aussi solubiliser les dérivés xanthiques (caféine, théophylline), l'acide $\beta$-glycyrrhétinique, l'acide asiatique, l'octopirox ou encore le rétinol et ses esters, les dérivés naturels de la famille des flavonoïdes. On peut encore utiliser dans la présente invention les agents modulant la différenciation et/ou la prolifération et/ou la pigmentation cutanée tels que la vitamine D et ses dérivés, les oestrogènes tels que l'oestradiol, l'acide kojique ou l'hydroquinone ; les agents anti-radicaux libres, tels que l'alpha-tocophérol ou ses esters, les superoxyde dismutases, certains chélatants de métaux ; des antagonistes de substance P et/ou de CGRP (Calcitonin Gene Related Peptide ou peptide lié au gène de la calcitonine) tels que l'Iris Pallida et les sels de strontium, notamment les chlorures et les nitrates de strontium, ou des antagonistes de substance P et/ou de CGRP tels que ceux décrits dans les demandes de brevet français FR-A-2719474 et FR-A-2729855.

Préférentiellement les actifs sont choisis parmi l'acide ascorbique et ses esters, l'acide kojique, l'acide citrique, l'acide caféique, l'acide salicylique, l'acide lactique, l'acide glycolique, l'acide malique, la dihydroxyacétone, l'alphatocophérol et ses esters.

Les actifs peuvent également être choisis parmi les enzymes, en particulier parmi les protéases.

Il est connu d'introduire dans les compositions cosmétiques et/ou dermatologiques des enzymes, et notamment des protéases utilisées pour leurs propriétés protéolytiques. Ces enzymes sont recherchées dans le domaine cosmétique pour leur pouvoir lissant et nettoyant, et leur aptitude à éliminer les cellules mortes de la peau.

Malheureusement, les enzymes présentent l'inconvénient d'être instables en milieu aqueux et d'être facilement dégradées ou modifiées sous l'influence de l'eau. Elles perdent ainsi rapidement de leur activité au cours du temps et cette instabilité va à l'encontre de l'efficacité recherchée.

Dans les compositions selon l'invention, lorsque l'actif est une enzyme, il a été constaté que celle-ci était stabilisée et conservait son activité au cours du temps.

De préférence, la proportion des différents composants doit être telle que la valeur d'activité en eau de la composition est inférieure ou égale à 0,7.

L'activité en eau $a_W$ d'un milieu contenant de l'eau est le rapport de la pression de vapeur d'eau du produit "$P_{H20}$ produit" et de la pression de vapeur de l'eau pure "$P_{H20}$ pur" à la même température. Elle peut être exprimée aussi comme le rapport du nombre de molécules d'eau "$N_{H20}$" sur le nombre de molécules totales "$N_{H20}$ + $N_{corps\ dissous}$", qui tient compte de celles des corps dissous "$N_{corps\ dissous}$".

Elle est donnée par les formules suivantes :

$$a_w = \frac{P_{H20\ produit}}{P_{H20\ pur}} = \frac{N_{H20}}{N_{H20\ +\ Ncorpsdissous}}$$

On peut utiliser différentes méthodes pour mesure l'activité en eau. La plus courante est la méthode manométrique par laquelle on mesure directement la pression de vapeur.

De manière classique, une composition cosmétique ou dermatologique a une activité en eau située autour de 0,95

à 0,99. Une activité en eau inférieure à 0,85 représente une diminution notable de l'activité en eau. Seules les compositions ayant une valeur d'activité en eau liée au plus égale à 0,85 permettent une bonne conservation de l'activité enzymatique des enzymes.

Les enzymes utilisées selon l'invention sont notamment les lactoperoxydases, les lipases, les protéases, les phospholipases et les cellulases.

La ou les enzymes utilisées selon l'invention sont de manière avantageuse une protéase. Elles peuvent être choisies par exemple parmi celle vendue sous la dénomination commerciale "Subtilisine SP 544" par la société Novo Nordisk et celle vendue sous la dénomination commerciale "Lysoveg" par la société Laboratoires Sérobiologiques de Nancy.

Dans la composition selon l'invention, l'enzyme peut être utilisée de manière avantageuse en une quantité allant de 0,001 à 15 % en poids, de préférence de 0,01 à 10 % en poids, et mieux de 0,05 à 5 % en poids par rapport au poids total de la composition.

Les compositions selon l'invention peuvent en outre contenir des charges insolubles : poudre de polyéthylène, particules de polyamide comme par exemple celles vendues sous la dénomination "ORGASOL" par la Société ATOCHEM également connues sous les noms (CTFA) de *"polyamide 12"* ou *"polyamide 6".* On peut également utiliser dans ces compositions du kaolin, des poudres de Nylon répertoriées sous le nom CTFA de *"Nylon 12"* ou *"Nylon 6".* De telles compositions sont intéressantes dans le nettoyage de peau pour leurs propriétés exfoliantes.

Les compositions selon l'invention ont la propriété de potentialiser les actifs qu'elles comprennent de telle sorte que ceux-ci ont la même efficacité dans les compositions selon l'invention après un très court temps d'application puis un rinçage, que dans une composition de soin qui est appliquée pendant plusieurs heures. Elle présentent l'avantage de proposer un meilleur confort que les compositions de soin non rincées. Le mode d'application permet d'obtenir un traitement non-thérapeutique de la peau plus uniforme par rapport aux compositions non rincées. Ce résultat est particulièrement avantageux dans le cas des traitements auto-bronzants, les compositions non-rincées présentant souvent le défaut de produire un bronzage irrégulier en raison d'une application non-uniforme, difficile à contrôler.

Les vecteurs selon l'invention ont l'apparence d'un gel, transparent, stable. En fonction des propriétés des actifs, en particulier leur facilité à être solubilisés dans le vecteur, on peut obtenir une composition de soin transparente, mais on peut aussi obtenir une composition de soin non transparente, en raison de l'insolubilité des actifs dans le vecteur ou parce qu'on lui a incorporé des composés qui lui retirent sa transparence, comme par exemple des agents nacrants ou des charges. En outre ces compositions ont une très bonne rinçabilité. En particulier, les esters gras d'hydrates de carbone en $C_5$ à $C_7$ confèrent à ces compositions une rinçabilité améliorée par rapport aux compositions à base d'esters de saccharose et celles à base d'esters de sorbitol.

L'invention a également pour objet l'utilisation d'une composition ayant l'aspect d'un gel transparent stable comprenant :

(i) au moins 20 % d'une phase grasse,
(ii) au moins esters gras d'hydrate de carbone en $C_5$-$C_7$,
(iii) au moins un polyol,

comme vecteur d'actif cosmétique.

Les compositions selon l'invention peuvent se présenter par exemple sous la forme d'un produit nettoyant et/ou démaquillant, d'un masque, d'un produit de gommage, d'un exfoliant.

**ESSAIS COMPARATIFS**

Un test cosmétique a été réalisé pour illustrer l'intérêt en termes d'efficacité et de confort des vecteurs selon l'invention avec comme actif, un mélange d'alpha-hydroxy-acides (acides lactique, glycolique, citrique, malique, tartrique). Un panel de 11 personnes a comparé une composition selon l'invention à un lait de nettoyage/démaquillage classique. Les deux formules contiennent le même taux d'actifs, introduits, selon un mode opératoire identique, dans le compartiment aqueux des compositions.

Pour chaque composition, les pourcentages sont donnés en poids de matière active par rapport au poids total de la composition.

Composition 1 (selon l'invention) :

| | |
|---|---|
| A) Phase aqueuse | |
| - Sesqui-stéarate de méthyl-glucose oxyéthyléné (20 OE) commercialisé sous la marque Glucamate SSE 20 | 4,5 % |
| - Glycérol | 10 % |
| - Mélange d'acides de fruits (acides glycolique, lactique, malique, tartrique) | 1 % |
| - Eau | 8 % |
| - Conservateurs | qs |
| B) Phase huileuse | |
| - Cyclométhicone | 18 % |
| - Myristate d'isopropyle | 12 % |
| - Isoparaffine | 30 % |
| - Isobutylène | 16 % |
| C) Parfum | 0,5 % |

Mode opératoire : le dérivé de glucose est dissous dans l'eau et les polyols sous agitation entre 25°C et 35°C jusqu'à l'obtention d'une pâte visqueuse homogène. La phase huileuse et le parfum sont alors introduits lentement sous forte agitation. Le produit est ensuite lissé à la pâle et débullé sous vide.

Composition 2 (selon l'art antérieur) :

| | |
|---|---|
| A) Phase aqueuse | |
| - Sesqui-stéarate de méthyl-glucose oxyéthyléné (20 OE) commercialisé sous la marque Glucamate SSE 20 | 2 % |
| - Sesqui-stéarate de méthyl-glucose commercialisé sous la marque Glucate SS | 2 % |
| - Glycérol | 4 % |
| - Mélange d'acides de fruits (acides glycolique, lactique, malique, tartrique) | 1 % |
| - Carbomer | 0,3 % |
| - conservateurs | 0,4 % |
| - Eau | qsp 100 |
| B) Phase huileuse | |
| - Alcool stéarylique | 1,3 % |
| - Cyclométhicone | 8 % |
| - Huile de sésame | 5 % |
| - Isobutylène | 3 % |
| C) Parfum | 0,2 % |

On prépare un lait démaquillant sous la forme d'une émulsion huile-dans-eau à partir de l'ensemble des composants ci-dessus.

La composition de l'exemple 1, comportant 76 % de phase grasse, et celle de l'exemple 2 comparatif, comportant

17,3 % de phase grasse, ont été testées sur un panel de 11 personnes de la manière suivante :

Les 11 personnes ont appliqué les 2 produits en hémi-visage, afin de bien comparer les effets ; chaque personne a effectué un temps de pose de 10 minutes avant de rincer le produit et de se sécher le visage.

Les utilisateurs ont été interrogés au sujet des inconforts ressentis pendant et après l'application, et sur les effets des deux compositions après application. Le résultat est le suivant :

- Inconforts : nombre d'utilisateurs s'étant plaints de rougeurs, picotements, échauffements, sécheresse de la peau, démangeaisons et brûlures :

  - avant rinçage

    . 7/11 personnes pour la formule de lait classique (composition 2),
    . 3/11 personnes avec la composition de l'invention (composition 1).

  - après rinçage :

    . 10/11 personnes pour la formule de lait classique (composition 2),
    . 2/11 personnes avec la composition selon l'invention (composition 1).

- Efficacité :

Peau plus uniforme :

réponse :

oui :

. 2/11 personnes pour la formule de lait classique (composition 2),
. 7/11 personnes avec la composition de l'invention (composition 1),

non :

. 6/11 personnes pour la formule de lait classique (composition 2),
. 2/11 personnes avec la composition de l'invention (composition 1).

Peau plus claire :

réponse :

oui :

. 3/11 personnes pour la formule de lait classique (composition 2),
. 4/11 personnes avec la composition de l'invention (composition 1),

non :

. 5/11 personnes pour la formule de lait classique (composition 2),
. 3/11 personnes avec la composition de l'invention (composition 1).

Peau plus douce :

réponse :

oui :

. 2/11 personnes pour la formule de lait classique (composition 2),
. 8/11 personnes avec la composition de l'invention (composition 1),

Ces essais comparatifs soulignent la supériorité des compositions selon l'invention par rapport aux compositions rinçables de l'art antérieur, à la fois en termes d'innocuité et d'efficacité.

## EXEMPLES DE COMPOSITIONS

Pour chaque composition, les pourcentages sont donnés en poids de matière active par rapport au poids total de la composition.
La viscosité est mesurée à 25°C à l'aide d'un viscosimètre Rhéomat 180 ; les mesures de viscosité sont données en Pascal seconde (Pa.s).

### Exemple 1

| | |
|---|---|
| A) Phase aqueuse | |
| - Di-oléate de méthylglucose oxyéthyléné (120 OE) | 3 % |
| - Sesqui-stéarate de méthyl-glucose oxyéthyléné (20 OE) | 1 % |
| - Glycérol | 15 % |
| - Acide glycolique | 2 % |
| - Eau | 4 % |
| B) Phase huileuse | |
| - Huile de vaseline | 10 % |
| - Palmitate d'éthyl-2-hexyle | 23,5 % |
| - Isoparaffine hydrogénée | 9 % |
| - Cyclopentadiméthylsiloxane | 14 % |
| - Tetraméthyl hexane-heptane-octane | 18,5 % |
| - Parfum | qs |

Mode opératoire : le dérivé de glucose est dissous dans l'eau et les polyols sous agitation entre 25°C et 35°C jusqu'à l'obtention d'une pâte visqueuse homogène. La phase huileuse est alors introduite lentement sous forte agitation. Le produit est ensuite lissé à la pâle et débullé sous vide.

Cette composition a un aspect gélifié transparent. cristallin, très attrayant d'un point de vue visuel. Elle a une viscosité Rhéomat de 9 Pa.s.

L'étalement des produits sur la peau est facile, la rinçabilité est excellente. La peau après rinçage est nette, d'un toucher soyeux, non gras.

**Exemple 2**

| A) Phase aqueuse : | |
|---|---|
| - Di-oléate de méthylglucose oxyéthyléné (120 OE) | 6 % |
| - Sesqui-stéarate de méthyl-glucose oxyéthyléné (20 OE) | 2 % |
| - Glycérol | 11 % |
| - PEG-20 | 6 % |
| - Dihydroxyacétone | 10 % |
| - Eau | 5 % |
| B) Phase grasse : | |
| - Huile de vaseline | 12 % |
| - Isoparaffine hydrogénée | 26 % |
| - Cyclométhicone | 22 % |

Cette composition a un aspect gélifié transparent, cristallin, très attrayant d'un point de vue visuel. Elle a une viscosité Rhéomat de 9 Pa.s.

Après un temps de pose de 10 min suivi d'un rinçage, on observe un hâle uniforme et sans taches. Une répétition quotidienne de ce traitement cosmétique permet de faire évoluer ce hâle de façon régulière en lui conférant l'aspect d'un bronzage naturel.

**Exemple 3 :**

| A) Phase aqueuse : | |
|---|---|
| - Sesquistéarate de méthyl-glucose oxyéthyléné (20 OE) commercialisé sous la marque Glucamate SSE 20 | 4,5 % |
| - Glycérol | 10 % |
| - Subtilisine SP544 | 0,1 % |
| - Eau | 8,5 % |
| - Conservateurs | qs |
| B) Phase huileuse | |
| - Cyclométhicone | 18 % |
| - Myristate d'isopropyle | 12 % |
| - Isoparaffine | 30 % |
| - Isobutylène | 16 % |
| C) Parfum | 0,5 % |

Cette composition a un aspect gélifié transparent, cristallin, très attrayant d'un point de vue visuel. Cette composition est apte à faciliter l'élimination des cellules de la peau et à éclaircir le teint.

**Revendications**

1. Composition cosmétique et/ou dermatologique comprenant au moins un actif cosmétique et/ou dermatologique

caractérisée en ce que cet actif est dissous ou dispersé dans un vecteur ayant l'aspect d'un gel transparent comprenant :

(i) au moins 20 % d'une phase grasse,
(ii) au moins un ester gras d'hydrate de carbone en $C_5$-$C_7$,
(iii) au moins un polyol.

**2.** Composition selon la revendication précédente, caractérisée en ce qu'elle comprend en outre de l'eau.

**3.** Composition selon l'une quelconque des revendications précédentes, caractérisée en ce que l'hydrate de carbone en $C_5$-$C_7$ est choisi parmi les pentoses, hexoses et heptoses et leurs dérivés alkyl-oloside dont le groupement alkyle contient de 1 à 6 atomes de carbone.

**4.** Composition selon l'une quelconque des revendications précédentes, caractérisée en ce que l'hydrate de carbone est le méthyl-1-glucoside.

**5.** Composition selon l'une quelconque des revendications précédentes, caractérisée en ce que l'ester gras d'hydrate de carbone en $C_5$-$C_7$ est un composé obtenu par réaction d'un acide gras à chaîne saturée ou insaturée ayant de 8 à 30 atomes de carbone et de préférence 12 à 22 atomes de carbone avec l'hydrate de carbone.

**6.** Composition selon l'une quelconque des revendications précédentes, caractérisée en ce que l'ester gras d'hydrate de carbone en $C_5$-$C_7$ est oxyalkyléné.

**7.** Composition selon l'une quelconque des revendications précédentes, caractérisée en ce que l'ester d'hydrate de carbone en $C_5$-$C_7$ est éthérifié par un ou plusieurs fragments oxyéthylène ou oxypropylène, l'ensemble des substituants oxyéthylène ou oxypropylène représentant de 5 à 200 unités oxyde d'alkylène.

**8.** Composition selon l'une quelconque des revendications précédentes, caractérisée en ce que l'ester gras d'hydrate de carbone est choisi parmi les composés répondant à la formule (I) :

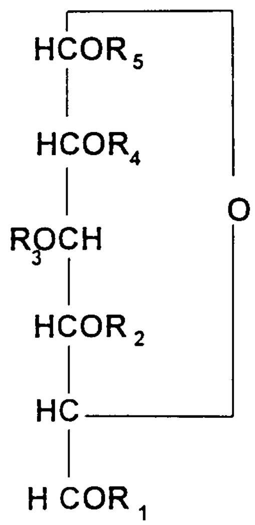

(I)

dans laquelle

$R_1$ est un groupement de formule (III) :

$$-OC-R' \quad (III)$$

R' étant choisi parmi les groupements alkyle linéaires ou ramifiés, saturés ou insaturés en $C_8$-$C_{30}$ et préférentiellement en $C_{12}$-$C_{22}$,

$R_2$, $R_3$, $R_4$, identiques ou différents, représentent un groupement choisi parmi :

- un atome d'hydrogène,
- un groupement de formule (II) :

$$-(C_nH_{2n}O)_p-R \quad (II)$$

avec n=2 ou 3 ; p est un entier allant de 2 à 50 ; R représente un atome d'hydrogène ou un groupement alkyle en $C_1$-$C_6$,
- un groupement de formule (III) :

$$-OC-R' \quad (III)$$

R' étant choisi parmi les groupements alkyle linéaires ou ramifiés, saturés ou insaturés en $C_8$-$C_{30}$ et préférentiellement en $C_{12}$-$C_{22}$,
l'un au moins de $R_2$, $R_3$, $R_4$ étant un groupement de formule (II), le total des résidus oxyalkylénés, $\Sigma p$, étant compris entre 5 et 200,

$R_5$ étant un groupement alkyle en $C_1$-$C_6$, linéaire ou ramifié, saturé ou insaturé.

**9.** Composition selon la revendication précédente, caractérisée en ce que

- $R_5$= $CH_3$,
- au moins deux groupements parmi $R_2$, $R_3$, $R_4$, répondent à la formule (II),
- $\Sigma p$ est compris entre 15 et 150,
- R=H,
- au moins un groupement parmi $R_1$, $R_2$, $R_3$, $R_4$, répond à la formule (III),
- R' est choisi parmi les groupements alkyle en $C_{16}$-$C_{20}$,
- aucun des groupements $R_2$, $R_3$, $R_4$, n'est l'atome d'hydrogène.

**10.** Composition selon l'une quelconque des revendications précédentes, caractérisée en ce qu'elle comprend, en poids par rapport au poids total de la composition, de 0,5 à 50% d'ester d'hydrate de carbone en $C_5$-$C_7$ et préférentiellement de 2 à 20%.

**11.** Composition selon l'une quelconque des revendications précédentes, caractérisée en ce que le polyol est choisi parmi l'éthylène glycol, le propylène glycol, le 1,3-butylène glycol, le dipropylène glycol, la glycérine, la diglycérine, la triglycérine et la tétraglycérine, le glucose, le maltose, le maltitol, le saccharose, le fructose, le sorbitol, les sucres issus de la décomposition de l'amidon et leurs mélanges.

**12.** Composition selon l'une quelconque des revendications précédentes, caractérisée en ce qu'elle comprend de 0,5 à 60% en poids par rapport au poids total de la composition, de préférence de 2 à 40% et encore plus préférentiellement de 5 à 30%, de polyol.

**13.** Composition selon l'une quelconque des revendications précédentes, caractérisée en ce que la phase grasse comprend une huile ou un mélange d'huiles ayant un indice de réfraction $^{20}n_D \leq 1{,}45$.

**14.** Composition selon l'une quelconque des revendications précédentes, caractérisée en ce que la phase grasse représente de 20 à 95% en poids du poids total de la composition, de préférence de 20 à 85%.

**15.** Composition selon l'une quelconque des revendications 2 à 14, caractérisée en ce que l'eau représente de 0,01 à 30%, avantageusement de 2 à 20% en poids par rapport au poids total de la composition.

**16.** Composition selon l'une quelconque des revendications 2 à 15, caractérisée en ce que l'eau est choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevard-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Uriage-les-bains, l'eau d'Avenne.

**17.** Composition selon l'une quelconque des revendications précédentes, caractérisée en ce que l'actif cosmétique et/ou dermatologique est choisi parmi les actifs dépigmentants, kératolytiques, amincissants, tenseurs, de traitement de l'acné, antiséborrhéiques, anti-inflammatoires. autobronzants, antiradicaux libres, raffermissants, les actifs traitant des maladies de peau comme les mycoses, les dermites, le psoriasis.

**18.** Composition selon l'une quelconque des revendications précédentes, caractérisée en ce que l'actif cosmétique et/ou dermatologique représente de 0,1 à 10% en poids par rapport au poids total de la composition.

**19.** Composition selon l'une quelconque des revendications précédentes, caractérisée en ce que l'actif cosmétique et/ou dermatologique est choisi parmi l'acide ascorbique et ses esters, l'acide kojique, l'acide citrique, l'acide caféique, l'acide salicylique, l'acide lactique, l'acide glycolique, l'acide malique, la dihydroxyacétone, l'alpha-tocophérol et ses esters.

**20.** Composition selon l'une quelconque des revendications 1 à 18, caractérisée en ce que l'actif cosmétique et/ou dermatologique est choisi parmi les enzymes.

**21.** Composition selon la revendication 20, caractérisée en ce que l'actif cosmétique et/ou dermatologique est choisi parmi : les lactoperoxydases, les lipases, les protéases , les phospholipases et les cellulases.

**22.** Composition selon l'une quelconque des revendications 20 et 21, caractérisée en ce que l'actif cosmétique et/ou dermatologique est choisi parmi les protéases.

**23.** Composition selon l'une quelconque des revendications 20 à 22, caractérisée en ce que l'enzyme est présente dans la composition en une quantité allant de 0,001 à 15 % en poids de préférence de 0,01 à 10 % en poids, par rapport au poids total de la composition.

**24.** Composition selon l'une quelconque des revendications 20 à 23, caractérisée en ce que son activité en eau est inférieure ou égale à 0,85.

**25.** Composition selon la revendication 24, caractérisée en ce que son activité en eau est inférieure ou égale à 0,7.

**26.** Composition selon l'une quelconque des revendications précédentes, caractérisée en ce qu'elle est sous la forme d'un produit nettoyant et/ou démaquillant, d'un masque, d'un produit de gommage, d'un exfoliant.

**27.** Utilisation d'une composition ayant l'aspect d'un gel transparent stable comprenant :

(i) au moins 20 % d'une phase grasse,
(ii) au moins un ester d'hydrate de carbone en C5-C7,
(iii) au moins un polyol,

comme vecteur d'actif cosmétique.

**28.** Utilisation selon la revendication précédente. caractérisée en ce que l'actif est une enzyme.

**29.** Utilisation selon la revendication précédente pour stabiliser l'enzyme.

**Claims**

**1.** Cosmetic and/or dermatological composition comprising at least one cosmetic and/or dermatological active agent, characterized in that this active agent is dissolved or dispersed in a vector having the appearance of a transparent gel, comprising:

(i) at least 20% of a fatty phase,
(ii) at least one fatty ester of a $C_5$-$C_7$ carbohydrate,
(iii) at least one polyol.

**2.** Composition according to the preceding claim, characterized in that it also comprises water.

3. Composition according to either of the preceding claims, characterized in that the $C_5$-$C_7$ carbohydrate is chosen from pentoses, hexoses and heptoses and their alkyl-oloside derivatives in which the alkyl group contains from 1 to 6 carbon atoms.

4. Composition according to any one of the preceding claims, characterized in that the carbohydrate is 1-methylglucoside.

5. Composition according to any one of the preceding claims, characterized in that the fatty ester of a $C_5$-$C_7$ carbohydrate is a compound obtained by reaction of a fatty acid containing a saturated or unsaturated chain having from 8 to 30 carbon atoms, and preferably 12 to 22 carbon atoms, with the carbohydrate.

6. Composition according to any one of the preceding claims, characterized in that the fatty ester of $C_5$-$C_7$ carbohydrate is oxyalkylenated.

7. Composition according to any one of the preceding claims, characterized in that the $C_5$-$C_7$ carbohydrate ester is etherified by one or more oxyethylene or oxypropylene fragments, the oxyethylene or oxypropylene substituents together representing from 5 to 200 alkylene oxide units.

8. Composition according to any one of the preceding claims, characterized in that the fatty ester of carbohydrate is chosen from compounds corresponding to formula (I):

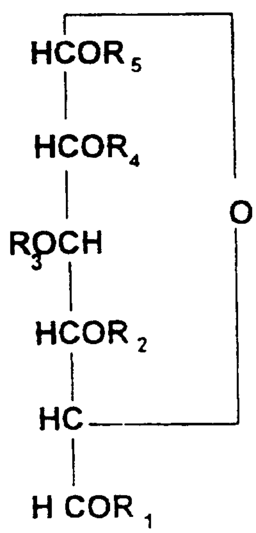

(I)

in which

$R_1$ is a group of formula (III):

$$-OC-R' \qquad (III)$$

R' being chosen from linear or branched, saturated or unsaturated $C_8$-$C_{30}$ and preferably $C_{12}$-$C_{22}$ alkyl groups,

$R_2$, $R_3$ and $R_4$, which may be identical or different, represent a group chosen from:

- a hydrogen atom,
- a group of formula (II):

$$-(C_nH_{2n}O)_p-R \qquad (II)$$

with n = 2 or 3; p is an integer ranging from 2 to 50; R represents a hydrogen atom or a $C_1$-$C_6$ alkyl group,

- a group of formula (III):

$$-OC-R' \quad (III)$$

R' being chosen from linear or branched, saturated or unsaturated $C_8$-$C_{30}$ and preferably $C_{12}$-$C_{22}$ alkyl groups,
at least one from among $R_2$, $R_3$ and $R_4$ being a group of formula (II), the total of the oxyalkylenated residues, $\Sigma p$, being between 5 and 200,

$R_5$ being a linear or branched, saturated or unsaturated $C_1$-$C_6$ alkyl group.

**9.** Composition according to the preceding claim, characterized in that

- $R_5 = CH_3$,
- at least two groups from among $R_2$, $R_3$ and $R_4$ correspond to formula (II),
- $\Sigma p$ is between 15 and 150,
- R = H,
- at least one group from among $R_1$, $R_2$, $R_3$ and $R_4$ corresponds to formula (III),
- R' is chosen from $C_{16}$-$C_{20}$ alkyl groups,
- none of the groups $R_2$, $R_3$ or $R_4$ is a hydrogen atom.

**10.** Composition according to any one of the preceding claims, characterized in that it comprises, by weight relative to the total weight of the composition, from 0.5 to 50% of $C_5$-$C_7$ carbohydrate ester and preferably from 2 to 20%.

**11.** Composition according to any one of the preceding claims, characterized in that the polyol is chosen from ethylene glycol, propylene glycol, 1,3-butylene glycol, dipropylene glycol, glycerol, diglycerol, triglycerol and tetraglycerol, glucose, maltose, maltitol, sucrose, fructose, sorbitol, sugars derived from the decomposition of starch and their mixtures.

**12.** Composition according to any one of the preceding claims, characterized in that it comprises from 0.5 to 60% by weight, relative to the total weight of the composition, preferably from 2 to 40% and even more preferably from 5 to 30%, of polyol.

**13.** Composition according to any one of the preceding claims, characterized in that the fatty phase comprises an oil or a mixture of oils having a refractive index $^{20}n_D \leq 1.45$.

**14.** Composition according to any one of the preceding claims, characterized in that the fatty phase represents from 20 to 95% by weight relative to the total weight of the composition, preferably from 20 to 85%.

**15.** Composition according to any one of Claims 2 to 14, characterized in that the water represents from 0.01 to 30%, advantageously from 2 to 20%, by weight relative to the total weight of the composition.

**16.** Composition according to any one of Claims 2 to 15, characterized in that the water is chosen from eau de Vittel, waters from the Vichy basin, eau d'Uriage, eau de la Roche Posay, eau de la Bourboule, eau d'Enghien-les-Bains, eau de Saint Gervais-les-Bains, eau de Néris-les-Bains, eau d'Allevard-les-Bains, eau de Digne, eau de Maizières, eau de Neyrac-les-Bains, eau de Lons-le-Saunier, les Eaux Bonnes, eau de Rochefort, eau de Saint Christau, eau des Fumades, eau de Tercis-les-bains, eau d'Uriage-les-bains and eau d'Avenne.

**17.** Composition according to any one of the preceding claims, characterized in that the cosmetic and/or dermatological active agent is chosen from depigmenting, keratolytic, slimming, tonifying, anti-acne, antiseborrheic, anti-inflammatory, self-tanning, anti-free-radical and firming active agents and active agents for treating skin diseases such as mycosis, dermatitis and psoriasis.

**18.** Composition according to any one of the preceding claims, characterized in that the cosmetic and/or dermatological active agent represents from 0.1 to 10% by weight relative to the total weight of the composition.

**19.** Composition according to any one of the preceding claims, characterized in that the cosmetic and/or dermatological active agent is chosen from ascorbic acid and its esters, kojic acid, citric acid, caffeic acid, salicylic acid, lactic

acid, glycolic acid, malic acid, dihydroxyacetone and α-tocopherol and its esters.

20. Composition according to any one of Claims 1 to 18, characterized in that the cosmetic and/or dermatological active agent is chosen from enzymes.

21. Composition according to Claim 20, characterized in that the cosmetic and/or dermatological active agent is chosen from lactoperoxidases, lipases, proteases, phospholipases and cellulases.

22. Composition according to either of Claims 20 and 21, characterized in that the cosmetic and/or dermatological active agent is chosen from proteases.

23. Composition according to any one of Claims 20 to 22, characterized in that the enzyme is present in the composition in an amount ranging from 0.001 to 15% by weight, preferably from 0.01 to 10% by weight, relative to the total weight of the composition.

24. Composition according to any one of Claims 20 to 23, characterized in that its water activity is less than or equal to 0.85.

25. Composition according to Claim 24, characterized in that its water activity is less than or equal to 0.7.

26. Composition according to any one of the preceding claims, characterized in that it is in the form of a cleansing and/or make-up-removing product, a mask, a scrubbing product or an exfoliant.

27. Use of a composition having the appearance of a stable transparent gel, comprising:

(i) at least 20% of a fatty phase,
(ii) at least one $C_5$-$C_7$ carbohydrate ester,
(iii) at least one polyol,

as vector for a cosmetic active agent.

28. Use according to the preceding claim, characterized in that the active agent is an enzyme.

29. Use according to the preceding claim in order to stabilize the enzyme.

**Patentansprüche**

1. Kosmetische und/oder dermatologische Zusammensetzung, die mindestens einen kosmetischen und/oder dermatologischen Wirkstoff enthält, dadurch gekennzeichnet, daß dieser Wirkstoff in einem Träger gelöst oder dispergiert ist, der das Aussehen eines transparenten Gels aufweist, mit:

(i) mindestens 20 % einer Fettphase,
(ii) mindestens einem $C_{5\text{-}7}$-Kohlenhydrat-Fettsäureester und
(i) mindestens einem Polyol.

2. Zusammensetzung nach dem vorhergehenden Anspruch, dadurch gekennzeichnet, daß sie ferner Wasser enthält.

3. Zusammensetzung nach einem der vorhergehenden Ansprüche, dadurch gekennzeichnet, daß das $C_{5\text{-}7}$-Kohlenhydrat unter den Pentosen, Hexosen und Heptosen und ihren Alkylglykosid-Derivaten, deren Alkylgruppe 1 bis 6 Kohlenstoffatome aufweist, ausgewählt ist.

4. Zusammensetzung nach einem der vorhergehenden Ansprüche, dadurch gekennzeichnet, daß das Kohlenhydrat das 1-Methyl-glucosid ist.

5. Zusammensetzung nach einem der vorhergehenden Ansprüche, dadurch gekennzeichnet, daß der $C_{5\text{-}7}$-Kohlenhydrat-Fettsäureester eine Verbindung ist, die durch Umsetzung einer Fettsäure mit gesättigter oder ungesättigter Kette, die 8 bis 30 Kohlenstoffatome und vorzugsweise 12 bis 22 Kohlenstoffatome aufweist, mit einem Kohlenhydrat hergestellt ist.

6. Zusammensetzung nach einem der vorhergehenden Ansprüche, dadurch gekennzeichnet, daß der $C_5$-$_7$-Kohlenhydrat-Fettsäureester alkoxyliert ist.

7. Zusammensetzung nach einem der vorhergehenden Ansprüche, dadurch gekennzeichnet, daß der $C_{5-7}$-Kohlenhydrat-Ester mit einem oder mehreren Ethylenoxid- oder Propylenoxidfragmenten verethert ist, wobei die gesamten Ethylenoxid- oder Propylenoxid-Substituenten 5 bis 200 Alkylenoxideinheiten ausmachen.

8. Zusammensetzung nach einem der vorhergehenden Ansprüche, dadurch gekennzeichnet, daß der Kohlenhydrat-Fettsäureester unter den Verbindungen der Formel (I) ausgewählt ist:

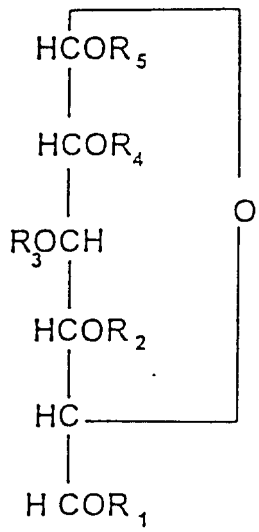

(I),

worin bedeuten:

$R_1$ eine Gruppe der Formel (III):

-OC-R' (III),

wobei R' unter den geradkettigen oder verzweigten, gesättigten oder ungesättigten Alkylgruppen mit 8 bis 30 Kohlenstoffatomen und vorzugsweise mit 12 bis 22 Kohlenstoffatomen ausgewählt ist,

$R_2$, $R_3$ und $R_4$, die identisch oder voneinander verschieden sind, eine Gruppe, die ausgewählt ist unter:

- Wasserstoff,
- einer Gruppe der Formel (II):

$$-(C_nH_{2n}O)_p-R \quad \text{(II)},$$

worin bedeuten: n = 2 oder 3; p eine ganze Zahl im Bereich von 2 bis 50; R Wasserstoff oder eine $C_{1-6}$-Alkylgruppe;
- einer Gruppe der Formel (III):

-OC-R' (III),

wobei R' unter den geradkettigen oder verzweigten, gesättigten oder ungesättigten Alkylgruppen mit 8 bis 30 Kohlenstoffatomen und vorzugsweise mit 12 bis 22 Kohlenstoffatomen ausgewählt ist,

wobei mindestens eine der Gruppen $R_2$, $R_3$ und $R_4$ eine Gruppe der Formel (II) bedeutet und wobei die

Gesamtheit der alkoxylierten Reste ($\Sigma p$) im Bereich von 5 bis 200 liegt,
$R_5$ eine geradkettige oder verzweigte, gesättigte oder ungesättigte $C_{1-6}$-Alkylgruppe.

**9.** Zusammensetzung nach dem vorhergehenden Anspruch, dadurch gekennzeichnet, daß

- $R_5 = CH_3$,
- mindestens zwei der Gruppen $R_2$, $R_3$ und $R_4$ der Formel (II) entsprechen,
- $\Sigma p$ im Bereich von 15 bis 150 liegt,
- R = H,
- mindestens einer der Gruppen $R_1$, $R_2$, $R_3$ und $R_4$ der Formel (III) entspricht,
- R' unter den $C_{16-20}$-Alkylgruppen ausgewählt ist,
- keine der Gruppen $R_2$, $R_3$ und $R_4$ Wasserstoff bedeutet.

**10.** Zusammensetzung nach einem der vorhergehenden Ansprüche, dadurch gekennzeichnet, daß sie 0,5 bis 50 Gew.-% $C_{5-7}$-Kohlenhydratester und vorzugsweise 2 bis 20 Gew.-% $C_{5-7}$-Kohlenhydratester, bezogen auf das Gesamtgewicht der Zusammensetzung, enthält.

**11.** Zusammensetzung nach einem der vorhergehenden Ansprüche, dadurch gekennzeichnet, daß das Polyol unter Ethylenglykol, Propylenglykol, 1,3-Butylenglykol, Dipropylenglykol, Glycerin, Diglycerin, Triglycerin, Tetraglycerin, Glucose, Maltose, Maltit, Saccharose, Fructose, Sorbit, Zuckern, die aus der Zersetzung von Stärke stammen, und deren Gemischen ausgewählt ist.

**12.** Zusammensetzung nach einem der vorhergehenden Ansprüche, dadurch gekennzeichnet, daß sie 0,5 bis 60 Gew.-% Polyol, vorzugsweise 2 bis 40 Gew.-% Polyol und noch bevorzugter 5 bis 30 Gew.-% Polyol, bezogen auf das Gesamtgewicht der Zusammensetzung, enthält.

**13.** Zusammensetzung nach einem der vorhergehenden Ansprüche, dadurch gekennzeichnet, daß die Fettphase ein Öl oder ein Gemisch von Ölen enthält, das einen Brechungsindex $^{20}n_D \leq 1{,}45$ aufweist.

**14.** Zusammensetzung nach einem der vorhergehenden Ansprüche, dadurch gekennzeichnet, daß die Fettphase 20 bis 95 Gew.-% und vorzugsweise 20 bis 85 Gew.-% des Gesamtgewichts der Zusammensetzung ausmacht.

**15.** Zusammensetzung nach einem der Ansprüche 2 bis 14, dadurch gekennzeichnet, daß das Wasser 0,01 bis 30 Gew.-% und vorteilhaft 2 bis 20 Gew.-%, bezogen auf das Gesamtgewicht der Zusammensetzung, ausmacht.

**16.** Zusammensetzung nach einem der Ansprüche 2 bis 15, dadurch gekennzeichnet, daß das Wasser unter Wasser aus Vittel, Wasser aus dem Vichy-Becken, Wasser aus Uriage, Wasser aus Roche Posay, Wasser aus Bourboule, Wasser aus Enghien-les-Bains, Wasser aus Saint-Gervais-les-Bains, Wasser aus Néris-les-Bains, Wasser aus Allevard-les-Bains, Wasser aus Digne, Wasser aus Maizières, Wasser aus Neyrac-les-Bains, Wasser aus Lons-le-Saunier, Eaux Bonnes, Wasser aus Rochefort, Wasser aus Saint Christau, Wasser aus Fumades, Wasser aus Tercis-les-bains, Wasser aus Uriage-les-bains und Wasser aus Avenne ausgewählt ist.

**17.** Zusammensetzung nach einem der vorhergehenden Ansprüche, dadurch gekennzeichnet, daß der kosmetische und/oder dermatologische Wirkstoff unter depigmentierenden, keratolytischen, schlankmachenden und waschaktiven Wirkstoffen, Wirkstoffen zur Behandlung von Akne und gegen Sebborhoe, entzündungshemmenden und selbstbräunenden Wirkstoffen, Wirkstoffen gegen freie Radikale, straffenden Wirkstoffen und Wirkstoffen zur Behandlung von Hautkrankheiten, wie Mykosen, Dermatitis und Psoriasis, ausgewählt ist.

**18.** Zusammensetzung nach einem der vorhergehenden Ansprüche, dadurch gekennzeichnet, daß der kosmetische und/oder dermatologische Wirkstoff 0,1 bis 10 Gew.-%, bezogen auf das Gesamtgewicht der Zusammensetzung, ausmacht.

**19.** Zusammensetzung nach einem der vorhergehenden Ansprüche, dadurch gekennzeichnet, daß der kosmetische und/oder dermatologische Wirkstoff unter Ascorbinsäure und ihren Estern, Kojisäure, Citronensäure, Kaffeesäure, Salicylsäure, Milchsäure, Glykolsäure, Äpfelsäure, Dihydroxyaceton, $\alpha$-Tocopherol und seinen Estern ausgewählt ist.

**20.** Zusammensetzung nach einem der Ansprüche 1 bis 18, dadurch gekennzeichnet, daß der kosmetische und/oder

dermatologische Wirkstoff unter den Enzymen ausgewählt ist.

**21.** Zusammensetzung nach Anspruch 20, dadurch gekennzeichnet, daß der kosmetische und/oder dermatologische Wirkstoff ausgewählt ist unter den Lactoperoxidasen, Lipasen, Proteasen, Phospholipasen und Cellulasen.

**22.** Zusammensetzung nach einem der Ansprüche 20 und 21, dadurch gekennzeichnet, daß der kosmetische und/oder dermatologische Wirkstoff unter den Proteasen ausgewählt ist.

**23.** Zusammensetzung nach einem der Ansprüche 20 bis 22, dadurch gekennzeichnet, daß das Enzym in der Zusammensetzung in einem Mengenanteil im Bereich von 0,001 bis 15 Gew.-% und vorzugsweise von 0,01 bis 10 Gew.-%, bezogen auf das Gesamtgewicht der Zusammensetzung, vorliegt.

**24.** Zusammensetzung nach einem der Ansprüche 20 bis 23, dadurch gekennzeichnet, daß ihre Wasseraktivität höchstens 0,85 beträgt.

**25.** Zusammensetzung nach Anspruch 24, dadurch gekennzeichnet, daß ihre Wasseraktivität höchstens 0,7 beträgt.

**26.** Zusammensetzung nach einem der vorhergehenden Ansprüche, dadurch gekennzeichnet, daß sie in Form eines reinigenden Produkts und/oder eines Produkts zum Abschminken, einer Maske, eines abradierenden Produkts oder eines Exfoliationsmittels vorliegt.

**27.** Verwendung einer Zusammensetzung, die das Aussehen eines stabilen, transparenten Gels aufweist, mit

(i) mindestens 20 % einer Fettphase,
(ii) mindestens einem $C_{5-7}$-Kohlenhydrat-Ester und
(iii) mindestens einem Polyol

als Träger eines kosmetischen Wirkstoffs.

**28.** Verwendung nach dem vorhergehenden Anspruch, dadurch gekennzeichnet, daß der Wirkstoff ein Enzym ist.

**29.** Verwendung nach dem vorhergehenden Anspruch zur Stabilisierung des Enzyms.